(19) Europäisches Patentamt
European Patent Office
Office européen des brevets

(11) Numéro de publication : 0 502 020 B1

(12) **FASCICULE DE BREVET EUROPEEN**

(45) Date de publication du fascicule du brevet : **26.01.94 Bulletin 94/04**

(21) Numéro de dépôt : **90917107.6**

(22) Date de dépôt : **14.11.90**

(86) Numéro de dépôt international : **PCT/FR90/00811**

(87) Numéro de publication internationale : **WO 91/07403 30.05.91 Gazette 91/12**

(51) Int. Cl.$^5$ : **C07D 471/04,** C07D 491/14, C07D 498/14, A61K 31/47, A61K 31/535, // A61K31/00 , (C07D471/04, 221:00, 221:00, 471:00, 491:00, 498:00)

(54) **NOUVEAUX DERIVES DE BENZO [b] PHENANTHROLINES-1,7 LEUR PREPARATION ET LEUR APPLICATION EN THERAPEUTIQUE.**

(30) Priorité : **14.11.89 FR 8914900**

(43) Date de publication de la demande : **09.09.92 Bulletin 92/37**

(45) Mention de la délivrance du brevet : **26.01.94 Bulletin 94/04**

(84) Etats contractants désignés : **AT BE CH DE DK ES FR GB GR IT LI LU NL SE**

(56) Documents cités : **EP-A- 0 087 951**

(73) Titulaire : **PIERRE FABRE MEDICAMENT**
**45, Place Abel Gance**
**F-92100 Boulogne (FR)**

(72) Inventeur : **Bigg, Dennis**
**122, avenue de Lavaur**
**F-81100 Castres (FR)**
Inventeur : **Lhomme, Jean**
**Dom. Universit., Bât. 52, Chimie Recherche**
**B.P. 68**
**F-38402 Saint-Martin-d'Hères Cédex (FR)**
Inventeur : **Salez, Hervé**
**Dom. Universit., Bât. 52, Chimie Recherche**
**B.P. 68**
**F-38402 Saint-Martin-d'Hères Cédex (FR)**
Inventeur : **Wardani, Abderrahim**
**Dom. Universit., Bât. 52, Chimie Recherche**
**B.P. 68**
**F-38402 Saint-Martin-d'Hères Cédex (FR)**

(74) Mandataire : **Ahner, Francis et al**
**CABINET REGIMBEAU 26, avenue Kléber**
**F-75008 Paris (FR)**

EP 0 502 020 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet de nouveaux dérivés de benzo [b] phénanthrolines-1,7, leur préparation et leur application en thérapeutique.

Les composés de l'invention répondent à la formule générale I :

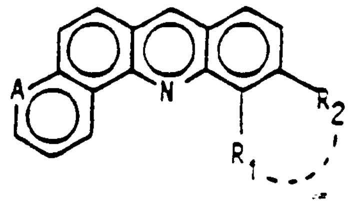

I

dans laquelle :

A représente un atome d'azote ou un radical Me-N$^{\oplus}$, la formule I devant être complétée dans ce dernier cas par un contranion X$^{\ominus}$ provenant d'un groupe X nucléofuge

$R_1$ représente, indépendamment de $R_2$,

- un radical formyle (CHO)
- un radical de formule $-CH_2OR_3$
  où $R_3$ est un atome d'hydrogène ou un groupe alkyle en $C_1$-$C_4$

$R_2$ représente, indépendamment de $R_1$,

- un radical hydroxyle
- un radical de formule $-NHR_4$ où $R_4$ est un atome d'hydrogène ou un groupe alkyle en $C_1$-$C_4$

$R_1$ et $R_2$ pris ensemble, représente :

- un radical $-CH_2OCH_2N(R_4)-$ où $R_4$ est un atome d'hydrogène ou un groupe alkyle en $C_1$-$C_4$
- un radical $-CH_2OCH_2O-$

L'invention couvre également les sels des composés de formule générale I avec des acides pharmaceutiquement acceptables.

Les composés de formule générale I de l'invention peuvent être préparés selon les schémas de réaction 1, 2 et 3 suivants :

Schéma 1

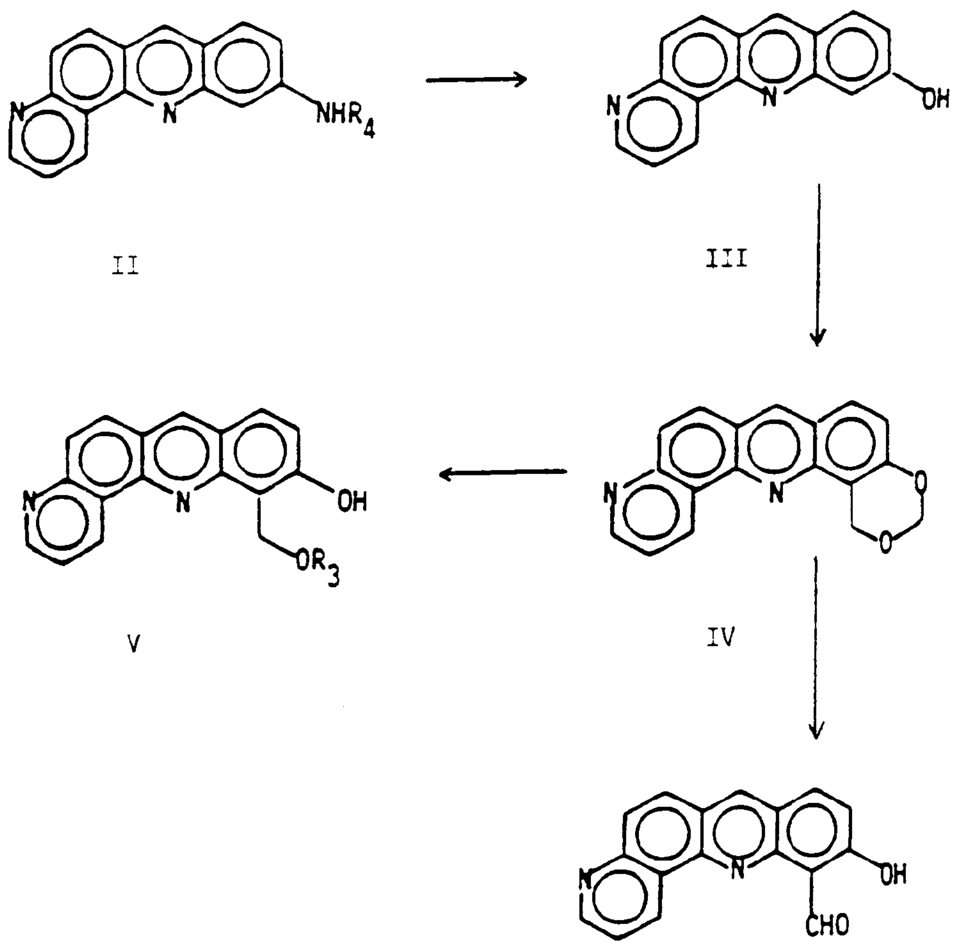

II

III

IV

V

VI

L'amino-10 benzo [b] phénanthroline-1,7 (formule II, $R_4$ = H) peut être obtenue selon des méthodes connues (A. Wardani. Thèse de Docteur en Chimie à l'Université des Sciences et Techniques de Lille, 18.07.86). Ce composé est transformé en dérivé diazonium par réaction avec un nitrite tel que le nitrite de sodium en milieu acide, par exemple, en milieu chlorhydrique.

Le sel de diazonium, de préférence le tétrafluoroborate, dérivé de l'amino-10 benzo [b] phénanthroline-1,7 est ensuite chauffé dans de l'acide aqueux, par exemple l'acide sulfurique 1N, pour fournir la hydroxy-10 benzo [b] phénanthroline-1,7 (formule III).

Le composé de formule III ainsi obtenu peut être transformé en tétrahydro-1,2,3,4 dioxa-2,4 napthto [5,6-b] phénanthroline-1,7 (formule IV) par réaction avec un excés de paraformaldéhyde en milieu acide. L'acide employé peut être un acide minéral ou, de préférence, un acide organique, par exemple l'acide méthane sulfonique.

Les produits de formule V où $R_3$ est un atome d'hydrogène ou un groupe alkyle en $C_1$-$C_4$ peuvent être obtenus à partir du produit de formule IV par chauffage de ce dernier avec un composé de formule $R_3OH$ en présence d'un acide, par exemple l'acide méthane sulfonique.

Le composé de formule VI peut être obtenu à partir du composé de formule IV par chauffage avec un acide aqueux en présence d'un oxydant tel que la dichloro-2,3 dicyano-5,6 benzoquinone-1,4. L'acide employé est de préférence un acide organique.

Schéma 2

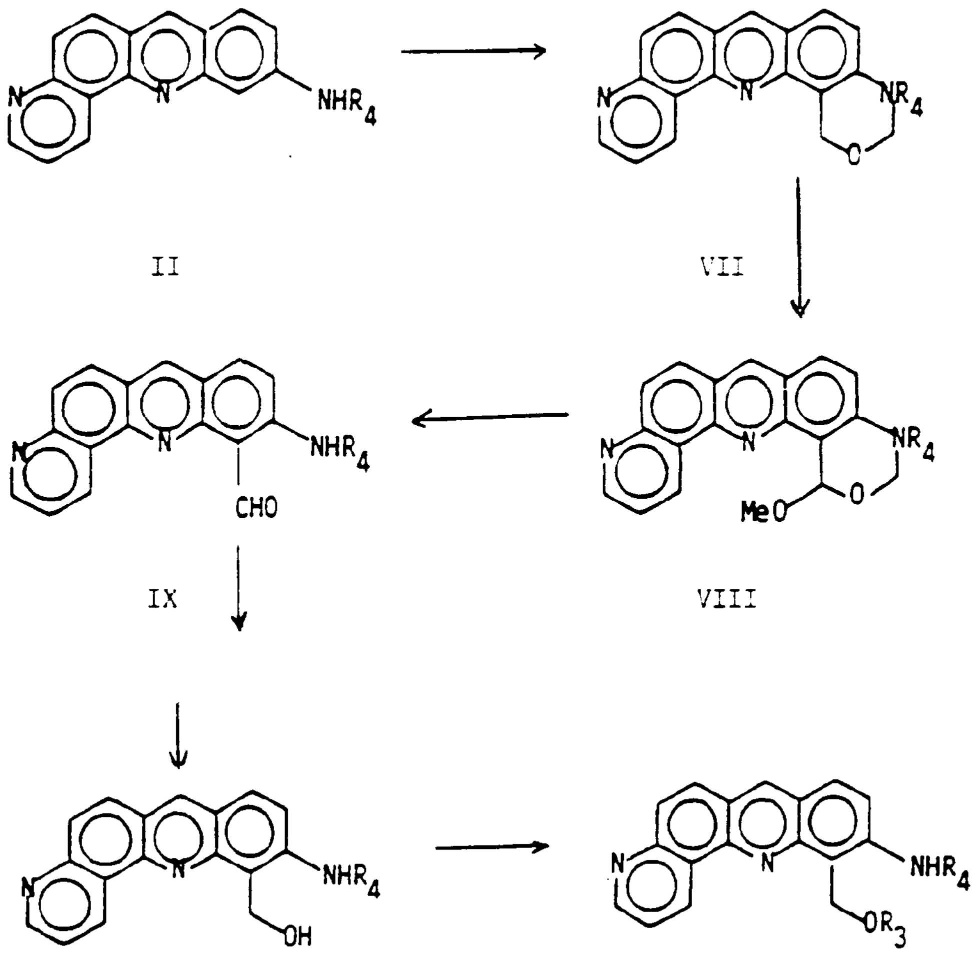

II VII VIII IX X XI

Les composés de formule II où $R_4$ représente un atome d'hydrogène ou un groupe alkyle en $C_1$-$C_4$ peuvent être préparés selon des méthodes décrites (A. Wardani, Thèse de Docteur en Chimie à l'Université des Sciences et Techniques de Lille, 18.07.86). Les dihydro-3,4 1H-benzoxazino [3,1] [5,6-b] phénanthrolines-1,7 de formule VII peuvent être préparées à partir des composés de formule II par réaction avec un excès de paraformaldéhyde en présence d'un acide minéral ou organique, par exemple, l'acide chlorhydrique ou l'acide méthane sulfonique.

Les oxazines de formule VII peuvent être transformées en composés de formule VIII par réaction avec un oxydant tel que la dichloro-2,3 dicyano-5,6 benzoquinone-1,4 dans le méthanol.

Un acétal de formule VIII peut être transformé en aldéhyde de formule IX par hydrolyse en milieu acide, utilisant soit un acide minéral tel que l'acide chlorhydrique, soit un acide organique, par exemple l'acide acétique.

Les produits de formule X peuvent être préparés à partir d'un aldéhyde de formule IX par des méthodes connues de réduction, par exemple l'utilisation du borohydrure de sodium dans un solvant alcoolique tel que le méthanol ou l'éthanol.

Les éthers de formule XI où $R_3$ représente un groupe alkyle en $C_1$-$C_4$ peuvent être preparés à partir des composés de formule X en traitant ces derniers avec un excès d'un alcool de formule $R_3OH$ en présence d'acide.

Schéma 3

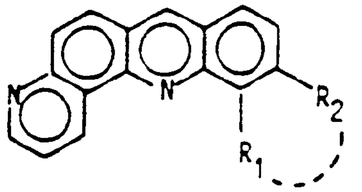

XII

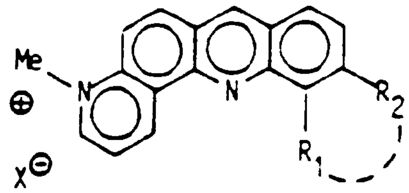

XIII

Les dérivés de formule XIII peuvent être obtenus à partir des composés de formule XII par réaction avec un produit de formule MeX, où X est un groupe nucléofuge au sein d'un solvant inerte, par exemple le toluène ou le benzène. L'agent de métnylation est, de préférence, employé en excès et peut être, à titre d'exemple, le sulfate de diméthyle.

Les exemples suivants illustrent l'invention.

Les analyses et les spectres IR et RMN confirment la structure des composés obtenus selon l'invention.

## Exemple 1

Tétrahydro-1,2,3,4 dioxa-2,4 naphto [5,6-b] phénanthroline-1,7

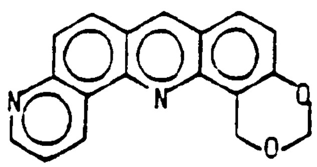

### 1a

On place l'amino-10 benzo [b] phénanthroline-1,7 (4 g, 16 mmol) dans 280 ml d'acide chlorhydrique 1N à 0°C. On ajoute le nitrite de sodium (2.8 g, 40 mmol) et on agite pendant 30 mn. On ajoute une solution de tétrafluoroborate de sodium (10 g, 84 mmol) dans 10 ml d'eau. On filtre le précipité jaune, on lave avec une solution de tétrafluoroborate de sodium, puis avec de l'eau, et ensuite avec de l'éther éthylique. Le sel de diazonium ainsi obtenu est utilisé directement pour l'étape suivante.
IR (KBr) : 3100, 2300, 1630, 1380, 1290, 1200, 1050, 930, 790 $cm^{-1}$

### 1b

Le sel de diazonium obtenu selon 1a (0,27 g) est chauffé à 80°C dans 30 ml d'acide sulfurique 1N pendant 30 mn. Le mélange réactionnel est alcalinisé avec l'ammoniac et extrait trois fois par le chloroforme. On concentre les extraits et on précipite la hydroxy-10 benzo [b] phénanthroline par l'addition de l'éther éthylique. Le produit est obtenu sous forme de monohydrate.
PF : 288°C

### 1c

A l'hydroxy-10 benzo [b] phénanthroline-1,7 (0,15 g, 0,6 mmol) obtenue selon 1b, on ajoute 4 ml d'acide méthane sulfonique. On ajoute un excès de paraformaldéhyde (0.06 g) et on agite à la température ambiante pendant 30 mn.

On verse le mélange réactionnel goutte à goutte dans un mélange chloroforme/ammoniac, on sépare et extrait la phase aqueuse deux fois par le chloroforme. Les phases organiques sont concentrées et la tétrahydro-1,2,3,4 dioxa-2,4 naphto [5,6-b] phénanthroline-1,7 est obtenue par précipitation avec l'éther de pétrole. Le produit fond à 219°C.

**Exemple 2**

Hydroxy-10 méthoxyméthyl-11 benzo [b] phénanthroline-1,7

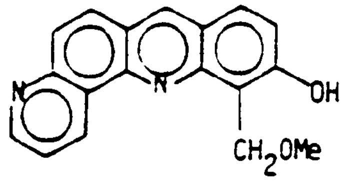

La tétrahydro-1,2,3,4 dioxa-2,4 naphto [5,6-b] phénanthroline-1,7 (0,12 g, 0,4 mmol), obtenue selon l'exemple 1, est dissoute dans un mélange méthanol/acide méthane sulfonique (1/1 : 2 ml). On agite la solution pendant 12 heures à 80°C. On laisse refroidir et on ajoute un mélange eau/bicarbonate de sodium/dichlorométhane. La phase organique est séchée et concentrée puis chromatographiée sur une colonne de silice en utilisant comme éluant de l'acétate d'ethyle. On obtient ainsi la hydroxy-10 méthoxyméthyl-11 benzo [b] phénanthroline-1,7 sous forme de cristaux jaunes.
PF : 145°C

**Exemple 3**

Formyl-11 hydroxy-10 benzo [b] phénanthroline-1,7

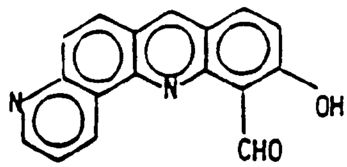

On ajoute la dichloro-2,3 dicyano-5,6 benzoquinone-1,4 (0,15 g, 0,7 mmol) à une solution de tétrahydro-1,2,3,4 dioxa-2,4 naphto [5,6-b] phénanthroline-1,7 (0.1 g. 0,3 mmol), obtenue selon l'exemple 1, dans un mélange d'acide formique/eau (9/1, 15 ml). On chauffe à reflux pendant 50 heures, on laisse refroidir et on filtre. Le filtrat est neutralisé par du bicarbonate de sodium, puis extrait avec du dichlorométhane. La phase organique est lavée deux fois à l'eau, séchée et évaporée à sec. Le résidu est chromatographié sur colonne de silice en utilisant l'acétate d'éthyle comme éluant. La formyl-11 hydroxy-10 benzo [b] phénanthroline-1,7 ainsi obtenue fond à 260°C.

**Exemple 4**

Dihydro-3,4 méthyl-4 1H-benzoxazino [3,1] [5,6-b] phénanthroline-1,7

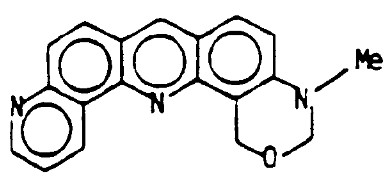

La méthylamino-10 benzo [b] phénanthroline-1,7 (1 g, 3,8 mmol) est ajoutée à 5 ml d'acide méthane sulfonique. On ajoute ensuite un excès de paraformaldéhyde (0,3 g) et on agite à température ambiante pendant 10 mn. La solution est alcalinisée avec de l'ammoniac puis extraite deux fois avec du dichlorométhane. La phase organique est lavée à l'eau, séchée et évaporée à sec. On obtient la dihydro-3,4 méthyl-4 1H-benzoxazino [3,1] [5,6-b] phénanthroline-1,7 sous forme de cristaux jaunes.
PF : 183°C

**Exemple 5**

Formyl-11 méthylamino-10 benzo [b] phénanthroline-1,7

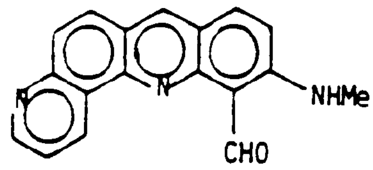

La dihydro-3,4 méthyl-4 1H-benzoxazino [3,1] [5,6-b] phénanthroline-1,7 (0,1 g, 0,3 mmol) obenue selon l'exemple 4, est dissoute dans 10 ml de méthanol. On ajoute ensuite un excès de dichloro-2,3 dicyano-5,6 benzoquinone-1,4 (0,2 g. 0,8 mmol) et on agite à la température ambiante pendant une heure. On ajoute ensuite 1 ml d'acide chlorhydrique 2N a la solution de dihydro-3,4 méthoxy-1 méthyl-4 1H-benzoxazino [3,1] [5,6-b] phénanthroline-1,7 (composé VIII, $R_4$=Me) ainsi formée et on agite pendant une heure.

Le mélange réactionnel est filtré puis neutralisé par le bicarbonate de sodium et enfin extrait avec du dichlorométhane. Après purification sur colonne de silice en utilisant comme éluant l'acétate d'éthyle on obtient la formyl-11 méthylamino-10 benzo [b] phénanthroline-1,7 qui fond à 260°C.

**Exemple 6**

Méthoxyméthyl-11 méthylamino-10 benzo [b] phénanthroline-1,7

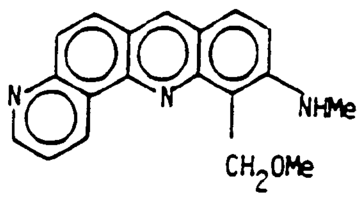

La formyl-11 méthylamino-10 benzo [b] phénanthroline-1,7 (0.2 g, 0,7 mmol) obtenue selon l'exemple 5 est dissoute dans 10 ml de méthanol.

Un excès de borohydrure de sodium est additionné à la solution puis le mélange est agité pendant une heure à la température ambiante.

On ajoute au mélange brut de l'alcool ainsi forme de l'acide chlorhydrique goutte à goutte jusqu'à apparition d'une coloration rouge.

Le mélange réactionnel est versé dans un mélange eau/bicarbonate de sodium/dichlorométhane. La phase organique est séparée, lavée deux fois à l'eau, séchée et évaporée. Après une purification sur colonne de silice en utilisant comme éluant un mélange acétate d'éthyle/dichlorométhane (7/3) on obtient la méthoxyméthyl-11 méthylamino-10 benzo [b] phénanthroline-1,7 sous forme de cristaux jaunes fondant à 160°C.

**Exemple 7**

Hydroxyméthyl-11 méthylamino-10 benzo [b] phénanthroline-1,7

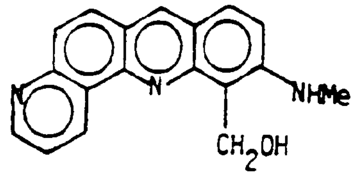

La formyl-11 méthylamino-10 benzo [b] phénanthroline-1,7 (0,16 g, 0,5 mmol) obtenue selon l'exemple 5 est dissoute dans 10 ml de méthanol. Un excès de borohydrure de sodium est additionné à la solution qui est laissée deux heures à la température ambiante. Le méthanol est éliminé par évaporation sous vide et le résidu est repris par un mélange eau/chloroforme. La phase organique est séparée, lavée deux fois à l'eau, puis séchée et évaporée. Le produit obtenu est purifié par chromatographie sur colonne de silice en utilisant comme éluant de l'acétate d'éthyle. On obtient ainsi l'hydroxyméthyl-11 méthylamino-10 benzo [b] phénanthroline-1,7 sous forme de cristaux oranges fondant à 190°C.

**Exemple 8**

Sulfate de méthyle du dihydro-3,4 diméthyl-4,10 1H-benzoxazino [3,1] [5,6-b] phénanthrolinium-1,7

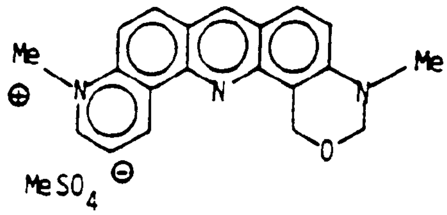

La dihydro-3,4 méthyl-4 1H-benzoxazino [3,1] [5,6-b] phénanthroline-1,7 (0,1 g, 0,3 mmol) obtenue selon l'exemple 4 est dissoute dans le toluéne (100 ml). On ajoute un excès de sulfate de diméthyle (0,2 ml, 2 mmol) et on agite pendant 30 heures à la température ambiante. Le précipité obtenu est filtré à travers une colonne d'alumine en utilisant comme éluant un mélange acétate d'éthyle/méthanol (7/3).
Les fractions pures sont réunies et concentrées sous pression réduite. On ajoute de l'éther éthylique pour précipiter le sulfate de méthyle du dihydro-3,4 diméthyl-4,10 1H-benzoxazino [3,1] [5,6-b] phénanthrolinium-1,7. PF : 270°C (décomposition).

Les composés de l'invention ont été soumis à des essais pharmacologiques qui ont montré leur intérêt en tant que cytotoxiques.

L'activité cytotoxique a été evaluée sur le modèle de la leucémie murine L 1210 cultivée en suspension en milieu iquide à 37°C sous une atmosphère de 5 % $CO_2$.

Les cellules en phase exponentielle de croissance sont incubées en présence de concentrations croissantes des produits.
Après 48 heures d'incubation, les cellules sont comptées (Coulter Counter) et l'effet cytotoxique est évalué par la détermination par régression linéaire de la concentration du produit ayant réduit de 50 % la croissance par rapport aux témoins ($IC_{50}$).

Les résultats obtenus sur certains composés de l'invention sont reportés, à titre d'exemple, dans le tableau suivant.

| Exemple N° | $IC_{50}$ (µM) |
|---|---|
| 2 | 6,5 |
| 4 | 1,3 |
| 6 | 1,1 |
| 7 | 0,17 |

Les composés de l'invention sont des cytotoxiques qui peuvent être utilisés en thérapeutique pour le traitement des cancers.

Les compositions pharmaceutiques peuvent être sous forme appropriée pour l'administration par voie orale, rectale, ou intraveineuse ; par exemple sous la forme de capsules, comprimés, granulés, gélules, solutions ou solutés liquides, et contenir les excipients appropriés.

## Revendications

1. Dérivés de benzo [b] phénanthrolines correspondant à la formule générale I

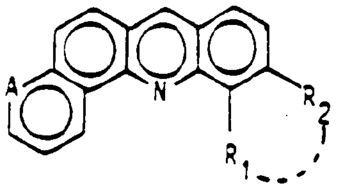

I

dans laquelle :

A représente un atome d'azote ou un radical Me-N$^{\oplus}$, la formule I devant être complétée dans ce dernier cas par un contranion $X^{\ominus}$ provenant d'un groupe X nucléofuge

$R_1$ représente, indépendamment de $R_2$,
- un radical formyle (CHO)
- un radical de formule $-CH_2OR_3$
  où $R_3$ est un atome d'hydrogène ou un groupe alkyle en $C_1$-$C_4$

$R_2$ représente, indépendamment de $R_1$,
- un radical hydroxyle
- un radical de formule $-NHR_4$ où $R_4$ est un atome d'hydrogène ou un groupe alkyle en $C_1$-$C_4$

ou $R_1$ et $R_2$ pris ensemble, représentent :
- un radical $-CH_2OCH_2N(R_4)-$ où $R_4$ est un atome d'hydrogène ou un groupe alkyle en $C_1$-$C_4$
- un radical $-CH_2OCH_2O-$

ainsi que les sels organiques ou minéraux thérapeutiquement acceptables de ces dérivés.

2. Dérivés selon la revendication 1 caractérisés en ce que $R_2$ représente un radical de formule $-NHR_4$ où $R_4$ est un groupe alkyle en $C_1$-$C_4$.

3. Dérivés selon la revendication 1 caractérisés en ce que $R_1$ représente un radical de formule $-CH_2OR_3$ où $R_3$ est un atome d'hydrogène ou un groupe alkyle en $C_1$-$C_4$.

4. Dérivés selon la revendication 1 caractérisés en ce que $R_1$ et $R_2$ pris ensemble représente un radical $-CH_2OCH_2N(R_4)$ où $R_4$ est un atome d'hydrogène ou un groupe alkyle en $C_1$-$C_4$.

5. Procédés de préparation de composés chimiques selon les revendications 1 et 3 caractérisé en ce que :
- on transforme le composé II ($R_4$=H) en composé III par réaction avec un nitrite tel que le nitrite de sodium en milieu acide pour obtenir un sel de diazonium que l'on chauffe en milieu acide aqueux

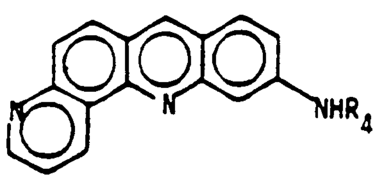

II

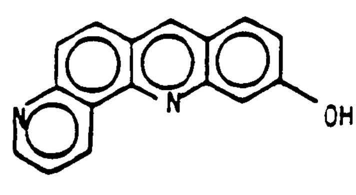

III

- on transforme le composé de formule III en composé de formule IV par réaction avec un excès de paraformaldéhyde en milieu acide ;

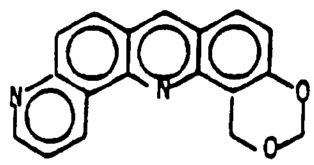

IV

- on chauffe le composé de formule IV avec un composé de formule $R_3OH$ où $R_3$ est un atome d'hydrogéne ou un groupe alkyle en $C_1$-$C_4$ en présence d'acide pour obtenir un produit de formule V où $R_3$ représente un atome d'hydrogène ou un groupe alkyle en $C_1$-$C_4$

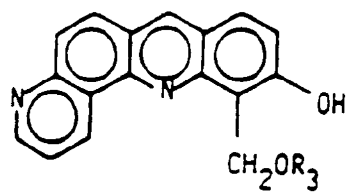

V

- on traite le composé IV avec un oxydant tel que la dichloro-2,3 dicyano-5,6 benzoquinone-1,4 en présence d'un acide aqueux pour obtenir un aldéhyde de formule VI

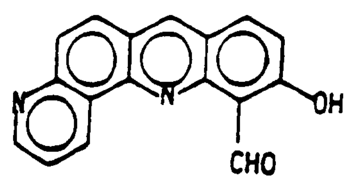

VI

6. Procédé de préparation de composés chimiques selon les revendications 1,2,3,4 caractérisé en ce que :
   - on fait réagir un composé de formule II où $R_4$ est un atome d'hydrogène ou un groupe alkyle en $C_1$-$C_4$ avec un excès de paraformaldéhyde en présence d'un acide pour obtenir un composé de formule VII

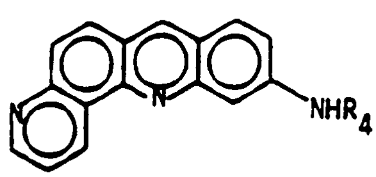

II

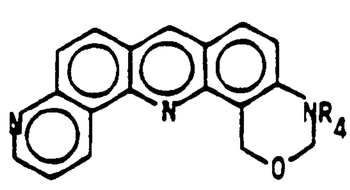

VII

   - on fait réagir le composé de formule VII ainsi obtenu avec un oxydant tel que la dichloro-2,3 dicyano-5,6 benzoquinone-1,4 dans le méthanol pour obtenir un acétal de formule VIII

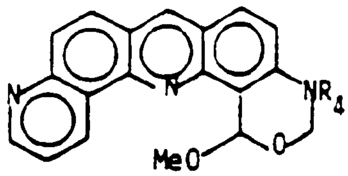

VIII

- on traite ensuite le composé de formule VIII avec de l'eau et de l'acide pour obtenir un aldéhyde de formule IX

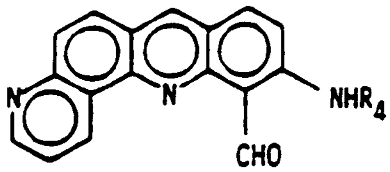

IX

- on fait réagir un aldéhyde de formule IX avec un agent de réduction tel que le borohydrure de sodium dans un solvant alcoolique tel que le méthanol ou l'éthanol pour obtenir un composé de formule X

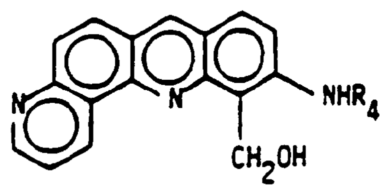

X

- on fait réagir un composé de formule X avec un excès d'un alcool de formule $R_3OH$, où $R_3$ est un groupe alkyle en $C_1$-$C_4$, en présence d'un acide pour obtenir un éther de formule XI où $R_3$ représente un groupe alkyle en $C_1$-$C_4$

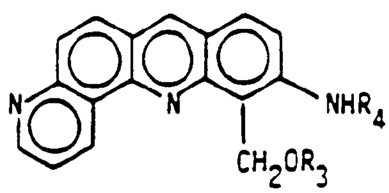

XI

7. Procédé de préparation de composés chimiques selon les revendications 1,2,3,4 caractérisé en ce que l'on fait réagir un composé de formule XII avec un agent de méthylation de formule MeX où X est un groupe nucléofuge, de préférence en excès dans un solvant inerte pour obtenir un composé de formule XIII

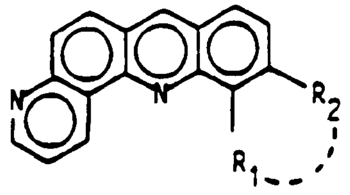

XII

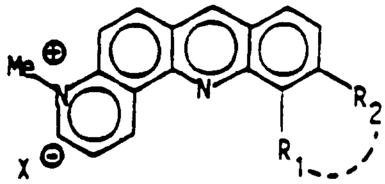

XIII

8. A titre de médicament nouveau, les dérivés de formule I selon l'une des revendications 1 à 4.

9. A titre de médicament anti-cancéreux les dérivés de formule I selon l'une des revendications 1 à 4.

10. Composition pharmaceutique, notamment destinée au traitement du cancer, caractérisée en ce qu'elle contient à titre de principe actif au moins un dérivé de formule générale I selon l'une des revendications 1 à 4.

## Patentansprüche

1. Benzo[b]phenanthrolinderivate der allgemeinen Formel I

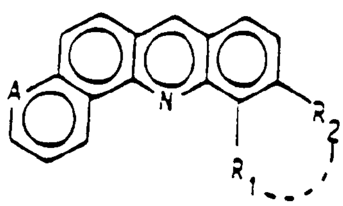

I

worin bedeuten:

| | |
|---|---|
| A | ein Stickstoffatom oder einen Rest Me-$N^+$, wobei die Formel I in letzterem Fall durch ein eine nucleofuge Gruppe X lieferndes Gegenanion $X^-$ ergänzt werden muß, |
| $R_1$ | unabhängig von $R_2$ einen Formylrest (CHO) oder einen Rest der Formel $-CH_2OR_3$ mit $R_3$ gleich einem Wasserstoffatom oder einer $C_1$ bis $C_4$-Alkylgruppe; |
| $R_2$ | unabhängig von $R_1$ einen Hydroxylrest oder einen Rest der Formel $-NHR_4$ mit $R_4$ gleich einem Wasserstoffatom oder einer $C_1$ bis $C_4$-Alkylgruppe oder |
| $R_1$ und $R_2$ | zusammengenommen einen Rest $-CH_2OCH_2N(R_4)-$ mit $R_4$ gleich einem Wasserstoffatom oder einer $C_1$ bis $C_4$-Alkylgruppe oder einen Rest $-CH_2OCH_2O-$ |

sowie deren therapeutisch akzeptable organische Salze oder Mineralsalze.

2. Derivate nach Anspruch 1, dadurch gekennzeichnet, daß $R_2$ für einen Rest der Formel $-NHR_4$ mit $R_4$ gleich einer $C_1$ bis $C_4$-Alkylgruppe steht.

3. Derivate nach Anspruch 1, dadurch gekennzeichnet, daß $R_1$ für einen Rest der Formel $-CH_2OR_3$ mit $R_3$ gleich einem Wasserstoffatom oder einer $C_1$ bis $C_4$-Alkylgruppe steht.

4. Derivate nach Anspruch 1, dadurch gekennzeichnet, daß $R_1$ und $R_2$ zusammengenommen für einen Rest $-CH_2OCH_2N(R_4)$ mit $R_4$ gleich einem Wasserstoffatom oder einer $C_1$ bis $C_4$-Alkylgruppe stehen.

5. Verfahren zur Herstellung chemischer Verbindungen nach Ansprüchen 1 und 3, dadurch gekennzeichnet, daß man die Verbindung II ($R_4$=H) durch Umsetzung mit einem Nitrit, z.B. Natriumnitrit, in saurem Milieu zur Bildung eines Diazoniumsalzes, das man in saurem wäßrigem Milieu erwärmt, in eine Verbindung III überführt;

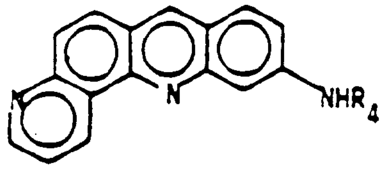

II

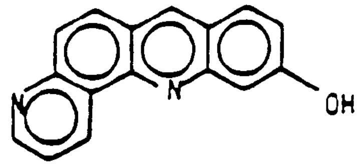

III

daß man die Verbindung der Formel III durch Umsetzung mit einem Überschuß an Paraformaldehyd in saurem Milieu in eine Verbindung der Formel IV überführt;

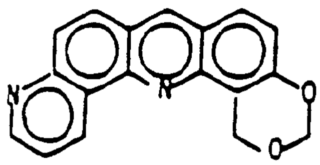

IV

daß man die Verbindung der Formel IV mit einer Verbindung der Formel $R_3OH$ mit $R_3$ gleich einem Wasserstoffatom oder einer $C_1$ bis $C_4$-Alkylgruppe in Gegenwart einer Säure zu einem Produkt der Formel V mit $R_3$ gleich einem Wasserstoffatom oder einer $C_1$ bis $C_4$-Alkylgruppe erwärmt;

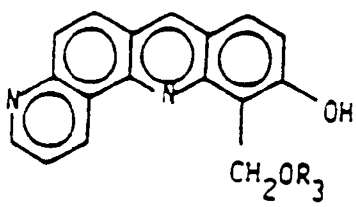

V

daß man die Verbindung IV in Gegenwart einer wäßrigen Säure mit einem Oxidationsmittel, z.B. Dichlor-2,3-dicyano-5,6-benzochinon-1,4, behandelt, um einen Aldehyd der Formel VI herzustellen.

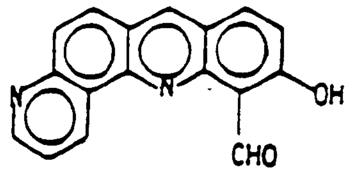

VI

6. Verfahren zur Herstellung chemischer Verbin dungen nach Ansprüchen 1, 2, 3, 4, dadurch gekennzeichnet, daß man eine Verbindung der Formel II mit $R_4$ gleich einem Wasserstoffatom oder einer $C_1$ bis $C_4$-Alkylgruppe in Gegenwart einer Säure mit einem Überschuß an Paraformaldehyd zu einer Verbindung der Formel VII reagieren läßt;

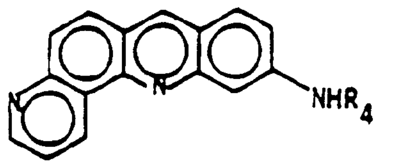

II

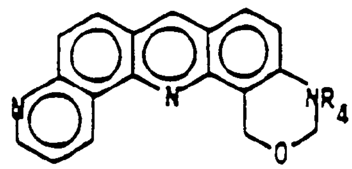

VII

daß man die hierbei erhaltene Verbindung der Formel VII in Methanol mit einem Oxidationsmittel, z.B. Dichlor-2,3-dicyano-5,6-benzochinon-1,4, zu einem Acetal der Formel VIII reagieren läßt;

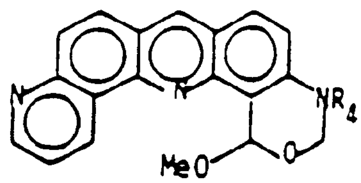

VIII

daß man anschließend die Verbindung der Formel VIII mit Wasser und einer Säure behandelt, um einen Aldehyd der Formel IX herzustellen;

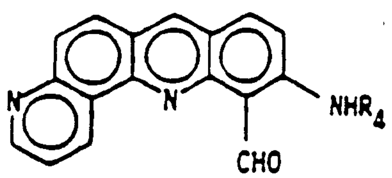

IX

daß man einen Aldehyd der Formel IX in einem alkoholischen Lösungsmittel, wie Methanol oder Ethanol mit einem Reduktionsmittel, wie Natriumborhydrid, zu einer Verbindung der Formel X reagieren läßt;

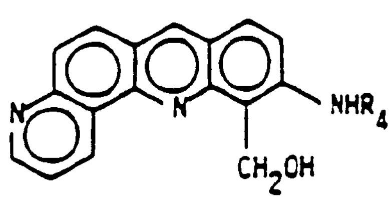

X

und daß man eine Verbindung der Formel X in Gegenwart einer Säure mit einem Überschuß an einem Alkohol der Formel $R_3OH$ mit $R_3$ gleich einer $C_1$ bis $C_4$-Alkylgruppe zu einem Ether der Formel XI mit $R_3$ gleich einer $C_1$ bis $C_4$-Alkylgruppe reagieren läßt.

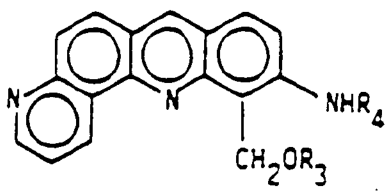

XI

7. Verfahren zur Herstellung chemischer Verbin dungen nach Ansprüchen 1, 2, 3, 4, dadurch gekennzeichnet, daß man eine Verbindung der Formel XII in einem inerten Lösungsmittel mit einem Methylierungsmittel der Formel MeX mit X gleich einer nucleofugen Gruppe, vorzugsweise im Überschuß, zu einer Verbindung der Formel XIII reagieren läßt:

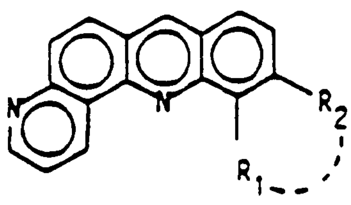

XII

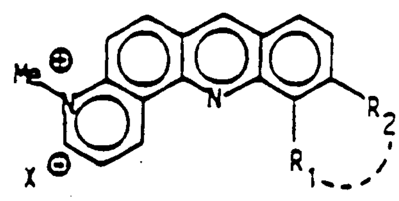

XIII

8. Derivate der Formel I nach einem der Anprüche 1 bis 4 als neues Arzneimittel.

9. Derivate der Formel I nach einem der Ansprüche 1 bis 4 als Arzneimittel gegen Krebs.

10. Arzneimittelzubereitung insbesondere zur Krebsbehandlung, dadurch gekennzeichnet, daß sie als aktiven Wirkstoff mindestens ein Derivat der allgemeinen Formel I nach einem der Ansprüche 1 bis 4 enthält.

## Claims

1. Derivatives of benzo[b]phenanthrolines corresponding to the general formula I

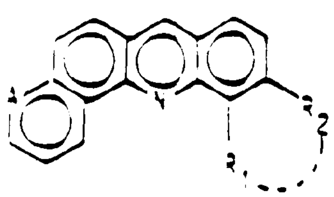

I

in which:

A denotes a nitrogen atom or a radical Me-N$^{\oplus}$, in this latter case the formula I having to be supplemented by a counteranion $X^{-}$ originating from a nucleophobic group X

$R_1$ denotes, independently of $R_2$,

- a formyl radical (CHO)
- a radical of formula $-CH_2OR_3$
  where $R_3$ is a hydrogen atom or a $C_1$-$C_4$ alkyl group

$R_2$ denotes, independently of $R_1$,

- a hydroxyl radical
- a radical of formula $-NHR_4$ where $R_4$ is a hydrogen atom or a $C_1$-$C_4$ alkyl group

or $R_1$ and $R_2$ taken together denote:

- a radical $-CH_2OCH_2N(R_4)-$ where $R_4$ is a hydrogen atom or a $C_1$-$C_4$ alkyl group
- a radical $-CH_2OCH_2O-$

and therapeutically acceptable organic or inorganic salts of these derivatives.

**2.** Derivatives according to Claim 1, characterized in that $R_2$ denotes a radical of formula $-NHR_4$ where $R_4$ is a $C_1$-$C_4$ alkyl group.

**3.** Derivatives according to Claim 1, characterized in that $R_1$ denotes a radical of formula $-CH_2OR_3$ where $R_3$ is a hydrogen atom or a $C_1$-$C_4$ alkyl group.

**4.** Derivatives according to Claim 1, characterized in that $R_1$ and $R_2$ taken together denotes a radical $-CH_2OCH_2N(R_4)$ where $R_4$ is a hydrogen atom or a $C_1$-$C_4$ alkyl group.

**5.** Processes for the preparation of chemical compounds according to Claims 1 and 3, characterized in that:

- the compound II ($R_4$=H) is converted into compound III by reaction with a nitrite such as sodium nitrite in acidic medium to obtain a diazonium salt which is heated in aqueous acidic medium

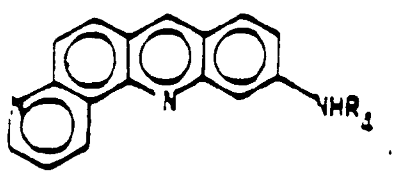

II

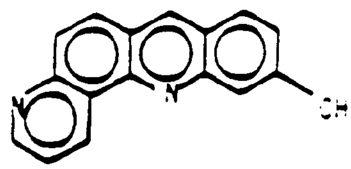

III

- the compound of formula III is converted into compound of formula IV by reaction with an excess of paraformaldehyde in acidic medium:

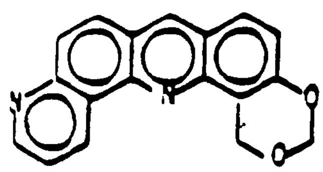

IV

- the compound of formula IV is heated with a compound of formula $R_3OH$ where $R_3$ is a hydrogen atom or a $C_1$-$C_4$ alkyl group in the presence of acid to obtain a product of formula V where $R_3$ denotes a hydrogen atom or a $C_1$-$C_4$ alkyl group

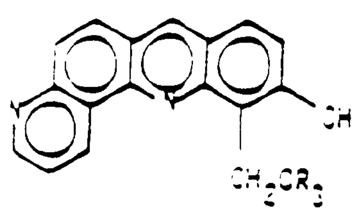

V

- the compound IV is treated with an oxidizing agent such as 2,3-dichloro-5,6-dicyano-1,4-benzoquinone in the presence of an aqueous acid to obtain an aldehyde of formula VI

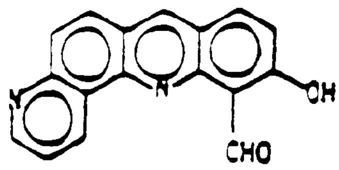

VI

6. Process for the preparation of chemical compounds according to Claims 1, 2, 3, 4, characterized in that:

- a compound of formula II where $R_4$ is a hydrogen atom or a $C_1$-$C_4$ alkyl group is reacted with an excess of paraformaldehyde in the presence of an acid to obtain a compound of formula VII

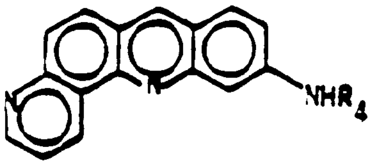

II

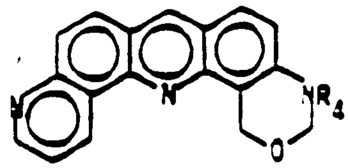

VII

- the compound of formula VII thus obtained is reacted with an oxidizing agent such as 2,3-dichloro-5,6-dicyano-1,4-benzoquinone in methanol to obtain an acetal of formula VIII

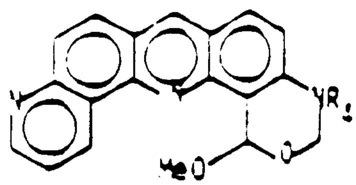

VIII

- the compound of formula VIII is then treated with water and acid to obtain an aldehyde of formula IX

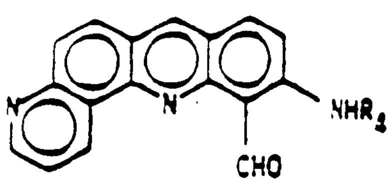

IX

- an aldehyde of formula IX is reacted with a reducing agent such as sodium borohydride in an alcoholic solvent such as methanol or ethanol to obtain a compound of formula X

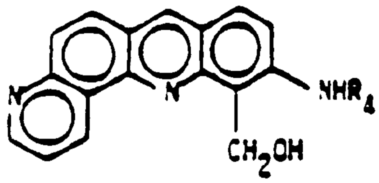

X

- a compound of formula X is reacted with an excess of an alcohol of formula $R_3OH$ where $R_3$ is a $C_1$-$C_4$ alkyl group, in the presence of an acid to obtain an ether of formula XI where $R_3$ denotes a $C_1$-$C_4$ alkyl group

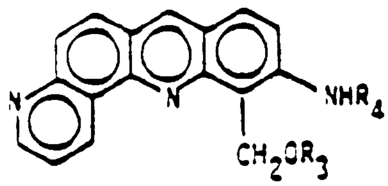

XI

7. Process for the preparation of chemical compounds according to Claims 1, 2, 3, 4, characterized in that a compound of formula XII is reacted with a methylating agent of formula MeX where X is a nucleophobic group, preferably in excess in an inert solvent to obtain a compound of formula XIII

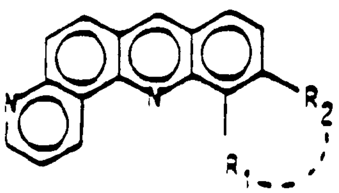

XII

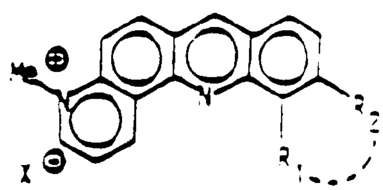

XIII

8. As new medication, the derivatives of formula I according to one of Claims 1 to 4.

9. As anticancer medication, the derivatives of formula I according to one of Claims 1 to 4.

10. Pharmaceutical composition, intended especially for the treatment of cancer, characterized in that it contains as active principle at least one derivative of general formula I according to one of Claims 1 to 4.